# EUROPEAN PATENT APPLICATION

(11) **EP 4 671 363 A1**
(43) Date of publication of application: **31.12.2025**
(21) Application number: 24760592.6
(22) Date of filing: 22.02.2024
(51) Int. Cl.: C12N 5/071, C12N 5/0735, C12N 5/074, C07K 14/54, A61K 35/545, A61P 29/00, A61P 13/12

(54) **APOPTOSIS-INDUCED STEM CELLS, PRODUCTION METHOD THEREFOR, AND COMPOSITION CONTAINING SAME FOR PREVENTING OR TREATING INFLAMMATORY OR RENAL DISEASES**

(30) Priority: 22.02.2023 KR 20230023549
(71) Applicant: Sungkwang Medical Foundation, Seoul 06135 (KR); Cha University Industry-Academic Cooperation Foundation, Pocheon-si, Gyeonggi-do 11160 (KR)
(72) Inventor: KANG, Eun Ju, Seoul 04595 (KR); LEE, Yeon Mi, Gwangju-si, Gyeonggi-do 12787 (KR); KANG, Soon-Suk, Seoul 05730 (KR); HWANG, Sae-Byeok, Seongnam-si, Gyeonggi-do 13506 (KR)
(74) Representative: Vossius & Partner Patentanwälte Rechtsanwälte mbB
(86) International application number: PCT/KR2024/002326
(87) International publication number: WO 2024/177403

(57) **Abstract**

The present disclosure relates to apoptosis-induced stem cells, a method for producing the same, and a pharmaceutical composition for the prevention or treatment of an inflammatory disease or a renal disease, including the same. Cells according to an aspect and a composition including the cells as an active ingredient have an increased IL-10 expression level without including a cryoprotectant, and thus can be effectively used for the prevention or treatment of an inflammatory disease or a renal disease.

## Description

### Technical Field

The present disclosure relates to apoptosis-induced stem cells, a method of producing the same, and a pharmaceutical composition for preventing or treating an inflammatory disease or a renal disease, including the same.

### Background Art

Stem cell therapeutic agents are medicines that use stem cells as a raw material and are administered to patients with diseases. These stem cells and stem cell therapeutic agents use viable cells as a raw material, and thus are sensitive to the environment and have a short shelf life.

Therefore, to preserve stem cells for a long period of time while maintaining the characteristics thereof, a cell cryopreservation process is essential. During cell cryopreservation, cryoprotectants are generally used because cells are damaged by crystallization of ice within the cells. However, there are few research reports on cryoprotectants that efficiently preserve stem cells for a long period of time and are harmless to the human body, and these reports are not standardized. Previously, substances such as dimethyl sulfoxide (DMSO) have been widely used as cryoprotectants for cryopreservation of various somatic cells including stem cells, reproductive cells such as fertilized eggs, oocytes and sperm, blood, and the like. However, DMSO interferes with cell survival and growth after freezing and thawing, making it unsuitable for mixing in a culture solution, and causes strong cytotoxicity when injected into living bodies in case that frozen stem cells are to be directly transplanted. In addition, cryoprotectants containing heterologous or animal-derived components, such as fetal bovine serum (FBS) and human serum, have a risk of causing infections with viruses, prions, and the like or immune responses, and furthermore, are not suitable for preservation of cell therapeutic agents used for treatment purposes.

Meanwhile, apoptosis is a type of programmed cell death that can be seen in multicellular organisms. Apoptosis is cell death resulting from changes in cell morphology and internal biochemical changes. Upon *in vivo* administration of stem cells, it is known that, as for adult stem cells, apoptosis of the administered stem cells is induced within one hour. Also, the administered stem cells are characterized in that most of the stem cells disappear from the body between 2 and 3 days.

Under these circumstances, there is a need for a safe cryopreserved stem cell therapeutic agent that can replace conventional stem cell therapeutic agents.

### Disclosure of Invention

### Technical Problem

An aspect is to provide an isolated apoptosis-induced cell that is induced to undergo apoptosis by cryopreservation and has a 1.5-fold or greater increase in interleukin-10 (IL-10) expression compared to the cell before cryopreservation.

Another aspect is to provide a pharmaceutical composition for the prevention or treatment of an inflammatory disease, including, as an active ingredient, an apoptosis-induced cell that is induced to undergo apoptosis by cryopreservation and has a 1.5-fold or greater increase in interleukin-10 (IL-10) expression compared to the cell before cryopreservation, or a culture solution of the cell.

Another aspect is to provide a method of preventing or treating an inflammatory disease, including administering the cell or a culture solution of the cell to a subject in need thereof.

Another aspect is to provide use of the cell or a cell solution of the cell for preparing a pharmaceutical composition for the prevention or treatment of an inflammatory disease.

Another aspect is to provide use of the cell or a culture solution of the cell for the prevention or treatment of an inflammatory disease.

Another aspect is to provide a method of preparing a composition containing apoptosis-induced cells, including preparing a cell-containing composition by immersing isolated cells in a solution containing a blood substitute, cryopreserving the cell-containing composition to prepare a composition containing apoptosis-induced cells, and thawing the composition containing apoptosis-induced cells.

### Solution to Problem

An aspect is to provide an isolated apoptosis-induced cell that is induced to undergo apoptosis by cryopreservation and has a 1.5-fold or greater increase in interleukin-10 (IL-10) expression compared to the cell before cryopreservation.

In an embodiment, the cell may be a stem cell.

The stem cell may be a totipotent stem cell, a pluripotent stem cell, or a multipotent stem cell.

The stem cell may be any one or more selected from the group consisting of an embryonic stem cell (ESC), an adult stem cell (ASC), and an induced pluripotent stem cell (iPSC).

The induced pluripotent stem cell is also referred to as an iPSC, and refers to a cell with pluripotency obtained by dedifferentiation from a differentiated cell (e.g., a somatic cell). The induced pluripotent stem cell may differentiate into various organ cells. The induced pluripotent stem cell may be obtained by reprogramming a differentiated cell by factors that induce reprogramming.

The adult stem cell may be derived from any one or more tissues selected from the group consisting of cord blood, fat, nerve, bone marrow, peripheral blood, muscle, liver, skin, placenta, amniotic membrane, and umbilical cord.

The adult stem cell may be any one or more types selected from the group consisting of a hematopoietic stem cell (HSC), a mesenchymal stem cell (MSC), a neural stem cell (NSC), a cardiac stem cell (CSC), an intestinal stem cell (ISC), a muscle stem cell (MuSC), a follicle stem cell (FSC), and an endothelial progenitor cell (EPC).

In an embodiment, the cell may be a lineage-restricted progenitor cell.

The lineage-restricted progenitor cell may be any one or more types selected from the group consisting of a hematopoietic progenitor cell, a mesenchymal progenitor cell, a neural progenitor cell, a cardiac progenitor cell, an intestinal progenitor cell, an adipose progenitor cell, a muscle progenitor cell, a hair follicle progenitor cell, and a vascular endothelial progenitor cell.

In an embodiment, the cell may be a differentiated somatic cell.

The differentiated somatic cell may be any one or more types selected from the group consisting of a hematopoietic cell, a mesenchymal cell, a neural cell, a cardiac cell, an intestinal cell, an adipocyte, a muscle cell, a hair follicle cell, and a vascular endothelial cell.

The term "stem cell" as used herein refers to a cell that possesses the ability to proliferate without substantial differentiation under certain circumstances and has the ability or potential to differentiate into a more specialized or differentiated phenotype under certain circumstances.

In an embodiment, the term progenitor cell or stem cell refers to a generalized parent cell whose progeny (descendants) specialize, usually in different directions, by acquiring completely individual characteristics through differentiation, such as in the progressive diversification of embryonic cells and tissues. Cell differentiation is a complex process that typically occurs through many cell divisions. Differentiated cells may be derived from pluripotent cells that themselves are derived from pluripotent cells, or the like. Although each of these pluripotent cells may be considered a stem cell, the range of cell types each can produce may vary considerably. Some differentiated cells also have the ability to generate cells with higher developmental potential. This ability may be natural or may be artificially induced upon treatment with various factors. In many biological cases, stem cells may be "multipotent" as stem cells can generate progeny of more than one distinct cell type, but this is not necessary for "stemness."

The term "differentiated cell" as used herein is a cell that has progressed further down the developmental pathway than a cell being considered. Thus, stem cells may differentiate into lineage-restricted progenitor cells (e.g., myocyte progenitor cells), which may eventually differentiate into other types of progenitor cells further down the pathway (e.g., myocyte progenitors), and then into terminally differentiated cells, such as myocytes, which perform characteristic roles in a given tissue type, and may or may not retain the ability to proliferate further.

In an embodiment, the cell may be cryopreserved and then thawed. The cryopreservation may refer to a process of preserving cells at a low temperature to preserve the cells for a long period of time while maintaining the characteristics of the cells. The thawing may refer to a process of restoring cryopreserved cells into active cells through a relatively high temperature for use according to the intended purpose.

The cells may be viable cells, with 75 % or greater of the entire population not having been induced to undergo apoptosis before cryopreservation. This may mean that at least 75% of the entire population is negative for Annexin V and PI. More specifically, at least 75 %, at least 80 %, at least 85 %, at least 90 %, at least 95 %, at least 98 %, at least 99 %, 100 %, 75 to 99 %, 80 to 99 %, 90 to 99 %, 75 to 95 %, 85 to 95 %, 75 to 90 % or 85 to 90 % of the entire population may be negative for Annexin V and PI.

The detection of Annexin V and PI may be derived using flow cytometry or fluorescence activated cell sorting (FACS) analysis of the cells.

The flow cytometry analysis may be single or multi-parameter flow cytometry, which may be a technique that performs multi-parameter analysis of single cells using single or multiple lasers. Each cell may be analyzed by visible light scattering or fluorescence emission that occurs when each laser rapidly passes through the cell. The fluorescence may be introduced through transfection and expression of common fluorescent proteins (e.g., GFP), or staining with fluorescently conjugated antibodies or fluorescent dyes.

The FACS may be a type of flow cytometry, which may be flow cytometry with an added function capable of sorting heterogeneously mixed cells on the basis of respective light scattering or fluorescence characteristics.

In this specification, the parameter in the flow cytometry may be Annexin V or propidium iodide (PI).

The term "positive" as used herein may mean that, in relation to apoptosis markers, the markers are present in greater amounts or higher concentrations compared to other non-apoptotic cells that serve as a reference. That is, a cell is considered positive for a marker in case that the cell can be distinguished from one or more other cell types using the marker because the marker is present inside or on the surface of the cell. It may also mean that the cell has the marker in an amount sufficient to produce a signal, for example, a signal from a flow cytometer, with a detection value greater than the background value.

The term "negative" as used herein may mean that, in relation to apoptosis markers, there is no difference in the markers compared to other non-apoptotic cells that serve as a reference. That is, it may mean that the markers cannot be detected compared to the background value.

The apoptosis may be cell death resulting from changes in cell morphology and internal biochemical changes. This may consist of cell swelling and rupture, changes in cell membrane, chromatin condensation and chromosome fragmentation, and phagocytosis by macrophages. Apoptosis may be divided into early apoptosis and late apoptosis. Early apoptosis may refer to a stage in which cells maintain morphology but have a flipped-off membrane structure with phosphatidylserine (PS) exposed to the outside of a cell, going through a complex cascade process, and late apoptosis may refer to a stage in which cell shrinking and membrane blebbing are observed.

In an embodiment, an apoptosis marker may be Annexin V or propidium iodide (PI). Annexin V may bind to phosphatidylserine (PS) on the cell membrane of apoptotic cells, and PI may bind to intracellular DNA in late apoptotic cells. The detection of Annexin V and PI may be derived through fluorescence analysis. The fluorescence analysis may be performed by flow cytometry.

In an embodiment, an Annexin V- and PI-negative cell may refer to a normal cell, an Annexin V-positive and PI-negative cell may refer to an early apoptotic cell, an Annexin V-positive and PI-positive cell may refer to a late apoptotic cell, and an Annexin V-negative and PI-positive cell may refer to a necrotic cell.

The cryopreservation may be performed at a temperature of -130 °C or -70°C or lower for 6 months or less or 6 months or longer. More specifically, the cryopreservation may be performed at a temperature of -60 °C to -80 °C, -65 °C to - 80 °C, -70 °C to -80 °C, -130 °C or lower, or -196 °C for 1 month to 12 months, 6 months to 12 months, 12 months or longer, 1 month to 24 months, 24 months or longer, 12 months to 36 months, 36 months or longer, 6 months or longer, 1 hour to 6 months, 12 hours to 6 months, 1 day to 6 months, 15 days to 6 months, 1 month to 6 months, 3 months to 6 months, 6 months or less, 6 months, 5 months, 4 months, 1 hour to 3 months, 12 hours to 3 months, 1 day to 3 months, 15 days to 3 months, 1 month to 3 months, 3 months or less, 3 months, 1 hour to 2 months, 12 hours to 2 months, 1 day to 2 months, 15 days to 2 months, 2 months or less, 2 months, 1 hour to 1 month, 12 hours to 1 month, 1 day to 1 month, 15 days to 1 month, 1 month or less, 1 month, 1 hour to 15 days, 12 hours to 15 days, 1 day to 15 days, 15 days or less, 15 days, 1 hour to 10 days, 12 hours to 10 days, 1 day to 10 days, 10 days or less, 10 days, 1 hour to 5 days, 12 hours to 5 days, 1 day to 5 days, 5 days or less, or 5 days.

The thawing may be performed at 0 to 5 °C for 10 minutes to 5 hours. More specifically, the thawing may be performed at a temperature of 0 to 10 °C, 0 to 2 °C, 0 to 4 °C, 0 to 6 °C, 0 to 8 °C, 2 to 3 °C, 2 to 5 °C, 2 to 8 °C, 3 to 5 °C, 3 to 7 °C, or 4 to 5 °C for 5 minutes to 5 hours, 10 minutes to 5 hours, 15 minutes to 5 hours, 30 minutes to 5 hours, 1 hour to 5 hours, 3 hours to 5 hours, 4 hours to 5 hours, 5 minutes to 30 minutes, 10 minutes to 30 minutes, 15 minutes to 30 minutes, 5 minutes to 20 minutes, 10 minutes to 20 minutes, 15 minutes to 20 minutes, 5 minutes to 1 hour, 10 minutes to 1 hour, 15 minutes to 1 hour, 30 minutes to 1 hour, 5 minutes to 3 hours, 10 minutes to 3 hours, 30 minutes to 3 hours, 1 hour to 3 hours, 5 minutes to 4 hours, 10 minutes to 4 hours, 30 minutes to 4 hours, 1 hour to 4 hours, or 3 hours to 4 hours.

In an embodiment, the cryopreservation may be performed without a cryoprotectant.

The cryoprotectant may be used to minimize damage to cells caused by ice crystals during cell cryopreservation. The cryoprotectant may refer to a compound such as dimethyl sulfoxide (DMSO), glycerol, 1,2-propane-diol, sucrose, methanol, glucose, proline, galactose, lactose, glycine betaine or fructose, or a substance containing an animal-derived component such as fetal bovine serum or human serum.

The cryoprotectant may interfere with cell survival and growth after freezing and thawing, making it unsuitable for mixing in a culture solution, and may cause strong cytotoxicity when injected into living bodies in case that cryopreserved stem cells are to be directly transplanted. Also, cryoprotectants containing animal-derived components may have a risk of causing infection with or immune responses to viruses, prions, and the like.

Cryopreservation performed without a cryoprotectant may use existing fluid as a solution. The solution may be existing fluid, and the existing fluid may refer to a blood substitute, replacement blood, or artificial blood.

The blood substitute, replacement blood or artificial blood may refer to a liquid used as a substitute for blood, and may refer to a preparation used for blood replenishment, plasma replenishment, maintenance of osmotic pressure, or the like. The terms "blood substitute," "replacement blood," or "artificial blood" may be used interchangeably.

The blood substitute, replacement blood or artificial blood may be any one selected from the group consisting of saline, 0.9 % saline, 9 % saline, sterile saline, Hartmann, Hartmann's solution, Hartmann's Dex, Hartmann's Dex solution, Hartmann D, Hartmann D solution, 5 % dextrose in saline, a sodium chloride solution, a 3 % sodium chloride solution, a sodium chloride-40 solution, a 0.45 % sodium chloride solution, 5 % dextrose with 0.45 % sodium chloride solution, a sodium chloride-dextrose solution, a sodium chloride-dextrose 1:3 (SD1:3) solution, a sodium chloride-dextrose 1:4 (SD1:4) solution, a sodium lactate-sodium chloride-dextrose 1:2:3 (LSD1:2:3) solution, a potassium chloride solution, a potassium chloride-20 solution, 5 % dextrose with potassium-sodium solution, a Patimasol solution, Plasma Solution A, a Plascon solution, a Halfsol solution, and a Plaju OP solution.

The blood substitute, replacement blood or artificial blood may include one or more components selected from the group consisting of sodium chloride, potassium chloride, calcium chloride, sodium lactate, glucose, dextrose (D-glucose), sodium gluconate, and sodium acetate.

Specifically, the saline may include sodium chloride, the Hartmann's solution may include sodium chloride, potassium chloride, calcium chloride, and sodium lactate, and the Hartmann's Dex solution may include sodium chloride, potassium chloride, calcium chloride, sodium lactate, and dextrose (D-glucose) (or glucose).

The concentration of the components in the blood substitute, replacement blood or artificial blood may be appropriately adjusted according to the purpose. Specifically, the saline may include 9 g/L of sodium chloride, the Hartmann's solution may include 6 g/L of sodium chloride, 300 mg/L of potassium chloride, 200 mg/L of calcium chloride, and 3.1 g/L of sodium lactate, and the Hartmann's Dex solution may include 6 g/L of sodium chloride, 300 mg/L of potassium chloride, 200 mg/L of calcium chloride, 6.2 g/L of sodium lactate, and 50 g/L of dextrose (D-glucose) (or glucose).

In an embodiment, the solution may refer to a solution including the cell and existing fluid or a blood substitute.

In an embodiment, the cells may be induced to undergo apoptosis after cryopreservation. The apoptosis may be induced by the fluid. In this regard, 90 % or greater of cells may be induced to undergo apoptosis by the fluid.

The cells induced to undergo apoptosis after cryopreservation may account for 75 % or greater of cells of the entire population. This may mean that, as a result of flow cytometry of the cells, at least 75 % of the entire population is positive for Annexin V. More specifically, at least 75 %, at least 80 %, at least 85 %, at least 90 %, at least 95 %, at least 98 %, at least 99 %, 75 to 99 %, 80 to 99 %, 100 %, 90 to 99 %, 75 to 95 %, 85 to 95 %, 75 to 90 %, or 85 to 90 % of the entire population may be positive for Annexin V.

The Hartmann's Dex solution may induce the highest level of early apoptosis. Early apoptotic cells may refer to cells that significantly retain the intrinsic functions thereof. The early apoptotic cells may have a longer shelf life than the late apoptotic cells.

The cells induced to undergo apoptosis after cryopreservation may refer to cells induced to undergo apoptosis after the thawing of the cryopreserved cells.

In an embodiment, the cells induced to undergo apoptosis after cryopreservation may have further increased expression of an immune or anti-inflammatory material after cryopreservation. The immune or anti-inflammatory material may refer to IL-10.

In an embodiment, the cells induced to undergo apoptosis after cryopreservation may have further increased expression of an immune or anti-inflammatory material during thawing after cryopreservation. The immune or anti-inflammatory material may refer to IL-10.

IL-10 may serve to regulate the proliferation and differentiation of various immune cells, including T cells, B cells, NK cells, antigen-presenting cells, mast cells, granulocytes, and the like, and to suppress inflammatory responses or modulate immune activation.

The expression of IL-10 may be increased 1.2 times or more, 1.3 times or more, 1.4 times or more, 1.5 times or more, 1.8 times or more, or 2.0 times or more, compared to the cell before cryopreservation. More specifically, the expression of IL-10 may be increased 1.5 times to 2.5 times, 1.2 times to 5.0 times, 1.5 times to 5.0 times, 2.0 times to 5.0 times, 2.5 times to 5.0 times, 3.0 times to 5.0 times, 1.2 times to 4.0 times, 1.5 times to 4.0 times, 2.0 times to 4.0 times, 2.5 times to 4.0 times, 3.0 times to 4.0 times, 1.2 times to 3.0 times, 1.3 times to 3.0 times, 1.5 times to 3.0 times, 1.8 times to 3.0 times, 2.0 times to 3.0 times, 2.2 times to 3.0 times, 2.5 times to 3.0 times, 1.2 times to 2.7 times, 1.3 times to 2.7 times, 1.5 times to 2.7 times, 1.8 times to 2.7 times, 2.0 times to 2.7 times, 2.2 times to 2.7 times, 2.5 times to 2.7 times, 1.2 times to 2.5 times, 1.3 times to 2.5 times, 1.5 times to 2.5 times, 1.8 times to 2.5 times, 2.0 times to 2.5 times, 2.2 times to 2.5 times, 1.2 times to 2.2 times, 1.3 times to 2.2 times, 1.5 times to 2.2 times, 1.8 times to 2.2 times, 2.0 times to 2.2 times, 1.2 times to 2.0 times, 1.3 times to 2.0 times, 1.5 times to 2.0 times, 1.8 times to 2.0 times, 1.2 times to 1.9 times, 1.3 times to 1.9 times, 1.5 times to 1.9 times, 1.8 times to 1.9 times, 1.2 times to 1.8 times, 1.3 times to 1.8 times, 1.5 times to 1.8 times, 1.2 times to 1.7 times, 1.3 times to 1.7 times, 1.5 times to 1.7 times, 1.2 times to 1.5 times, or 1.3 times to 1.5 times.

In an embodiment, the cells induced to undergo apoptosis after cryopreservation may have a preventive or therapeutic effect on an inflammatory disease or a renal disease.

The inflammatory disease may be, but is not limited to, any one selected from the group consisting of renal failure, nephritis, gastritis, inflammatory bowel disease, inflammatory respiratory disease, hepatitis, gastric ulcer, asthma, dermatitis, and enteritis.

The term "treatment" as used herein may refer to the healing of inflammatory diseases or renal diseases in a shortened time compared to natural healing. The treatment may include amelioration and/or alleviation of inflammatory diseases, renal diseases, or the like. Also, the treatment may mean healing and/or recovery from symptoms caused by inflammatory diseases, renal diseases, or the like.

The term "prevention" as used herein refers to a reduction in the occurrence of pathological cells or the degree of cell damage or loss in a subject. The prevention may be complete prevention or partial prevention. In this case, the prevention may refer to a phenomenon in which the occurrence of pathological cells, abnormal immune responses or the like in a subject is reduced compared to the case that the composition for the prevention or treatment of an inflammatory disease or a renal disease is not used.

In an embodiment, the cells induced to undergo apoptosis after cryopreservation may have no difference in distribution pattern after *in vivo* transplantation compared to the cell before cryopreservation. In other embodiments, the cells may have no difference in cell change pattern and migration pattern after *in vivo* transplantation compared to the cell before cryopreservation.

In an embodiment, the cells induced to undergo apoptosis after cryopreservation may have no difference in therapeutic effect after *in vivo* transplantation compared to the cell before cryopreservation.

Another aspect provides a composition for the amelioration, prevention or treatment of an inflammatory disease or a renal disease.

In an embodiment, the composition may include as an active ingredient: apoptosis-induced cells that are induced to undergo apoptosis by cryopreservation and have a 1.5-fold or greater increase in interleukin-10 (IL-10) expression compared to the cell before cryopreservation; or a culture solution of the cells.

Another aspect provides a pharmaceutical composition for the prevention or treatment of an inflammatory disease, including as an active ingredient: apoptosis-induced cells that are induced to undergo apoptosis by cryopreservation and have a 1.5-fold or greater increase in interleukin-10 (IL-10) expression compared to the cell before cryopreservation; or a culture solution of the cells.

Another aspect provides a method of preventing or treating an inflammatory disease, including administering the cells or a culture solution of the cells to a subject in need thereof.

Another aspect provides use of the cells or a culture solution of the cells for preparing a pharmaceutical composition for the prevention or treatment of an inflammatory disease.

Another aspect provides use of the cells or a culture solution of the cells for the prevention or treatment of an inflammatory disease.

The composition may include cells induced to undergo apoptosis after cryopreservation as an active ingredient.

The cells induced to undergo apoptosis after cryopreservation is as described above.

The inflammatory disease may be, but is not limited to, any one selected from the group consisting of renal failure, nephritis, gastritis, inflammatory bowel disease, inflammatory respiratory disease, hepatitis, gastric ulcer, asthma, dermatitis, and enteritis.

More specifically, the inflammatory disease may be any one selected from the group consisting of renal failure, nephritis, glomerulonephritis, gastritis, inflammatory bowel disease (IBD), hepatitis, inflammatory respiratory disease, gastric ulcer, irritable bowel syndrome, Behcet's disease, enteritis, Crohn's disease, asthma, ulcerative colitis, vasculitis, mucositis, stomatitis, peri-implantitis, periodontitis, pulpitis, gingivitis, pneumonia, dermatitis, atopic dermatitis, contact dermatitis, CREST syndrome, dermatitis herpetiformis, dermatomyositis, systemic scleroderma, erythema nodosum, Henoch-Schonlein purpura, Hidradenitis suppurativa, Lichen planus, Majeed syndrome, Schnitzler syndrome, psoriasis, eczema, acne, mouth ulcers, uveitis, pharyngitis, tonsillitis, otitis including otitis media, psoriatic arthritis, synovitis, meningitis, encephalitis, Bickerstaff's encephalitis, encephalomyelitis, spondylitis, osteomyelitis, Guillain-Barre syndrome, myelitis, neuromyelitis optica, cystitis, or acute inflammation at an infected site or wound site.

More specifically, the renal disease may be any one or more selected from the group consisting of chronic renal failure, acute renal failure, glomerulonephritis, interstitial nephritis, diabetic nephropathy, glomerulosclerosis, renal fibrosis, Alport syndrome, IDDM nephritis, mesangial proliferative glomerulonephritis, proliferative glomerulonephritis, crescentic glomerulonephritis, renal interstitial fibrosis, focal segmental glomerulosclerosis, membranous nephropathy, minimal change disease, pauci-immune rapidly progressive glomerulonephritis, IgA nephropathy, polycystic kidney disease, Dent's disease, nephrocytinosis, Heyman nephritis, nephrotic syndrome, renal ischemia, podocytopathy or podocyte disease, proteinuria, preeclampsia, renal lesion, collagen vascular disease, benign orthostatic (postural) proteinuria, IgM nephropathy, aminoaciduria, Fanconi syndrome, hypertensive nephrosclerosis, hemoglobinuria, myoglobinuria, Wegener's granulomatosis, decreased glomerular filtration rate (GFR), renal arterial sclerosis, lupus nephritis, proximal tubular dysfunction, acute renal transplant rejection, chronic kidney transplant rejection, and non-IgA mesangial proliferative glomerulonephritis.

The term "including as an active ingredient" means that the cells described herein are added to an extent that can exhibit the effects described above, and includes the meaning of being formulated in various forms by adding various components as secondary ingredients for drug delivery, stabilization, and the like.

In other embodiments, the composition may be a pharmaceutical composition or a cell therapeutic agent.

The pharmaceutical composition or cell therapeutic agent may further include a pharmaceutically acceptable diluent, carrier and/or additive. For example, the pharmaceutically acceptable diluent, carrier and/or additive may include sterilized water, physiological saline, buffers for common use (phosphoric acid, citric acid, or other organic acids), stabilizers, salts, antioxidants (such as ascorbic acid), surfactants, suspending agents, isotonic agents, preservatives, or the like. For local administration, the pharmaceutically acceptable diluent, carrier and/or additive may be used in combination with organic materials such as biopolymers, inorganic materials such as hydroxyapatite, specifically collagen matrix, polylactic acid polymers or copolymers, polyethylene glycol polymers or copolymers, chemical derivatives thereof, and the like.

In case that the cell therapeutic agent or pharmaceutical composition according to an embodiment is prepared in a dosage form suitable for injection, cell aggregates may be dissolved in a pharmaceutically acceptable carrier or may be cryopreserved in a dissolved solution state.

The pharmaceutical composition may be formulated as an oral or parenteral dosage form. The oral dosage form may be granules, powder, liquid, tablets, capsules, dry syrup, or a combination thereof. The parenteral dosage form may be an injection.

Another aspect provides a method of preparing a composition containing apoptosis-induced cells, including the steps of:
preparing a cell-containing composition by immersing isolated cells in a solution containing a blood substitute;
cryopreserving the cell-containing composition to prepare a composition containing apoptosis-induced cells; and
thawing the composition containing apoptosis-induced cells.

The cell-containing composition may have a preventive or therapeutic effect on an inflammatory disease or a kidney disease.

The cell-containing composition may be included as an active ingredient of a pharmaceutical composition or cell therapeutic agent.

The cells, cryopreservation, thawing, solution, cells induced to undergo apoptosis after cryopreservation, inflammatory disease, renal disease, pharmaceutical composition and cell therapeutic agent are as described above.

In an embodiment, the cells in the preparation of the cell-containing composition may be negative for both Annexin V and PI, and the cells in the thawing step may be positive for Annexin V.

The Annexin V, PI, positive and negative are as described above. Annexin V- and PI-negative cells may refer to normal cells, Annexin V-positive cells may refer to apoptotic cells, and Annexin V- and PI-positive cells may refer to late apoptotic cells.

Another aspect also provides a method of preventing, ameliorating or treating a condition of a subject, including treating a subject in need of the composition with an effective amount thereof or administering an effective amount of the composition to a subject in need thereof.

The condition of the subject may be a condition related to an inflammatory disease or renal disease. The inflammatory disease or the renal disease is as described above.

The pharmaceutical composition or cell therapeutic agent may be administered to a patient in a therapeutically effective amount or a pharmaceutically effective amount.

The terms "administering," "introducing," and "transplanting" are used interchangeably and may refer to the placement of a composition according to an embodiment into a subject by a method or route that results in at least partial localization of the composition according to an embodiment at a desired site. The composition according to an embodiment may be administered by any suitable route that delivers at least a portion of the cells or cellular components of the composition to a desired location in a surviving subject. The survival period of cells after administration to a subject may be as short as several hours, for example, 24 hours to several days, or as long as several years. The pharmaceutical composition may be administered to a patient in a therapeutically effective amount or a pharmaceutically effective amount.

The term "therapeutically effective amount" or "pharmaceutically effective amount" refers to an amount of a cell population or composition that is effective for preventing or treating an inflammatory disease or a renal disease, which is sufficient to treat the disease at a reasonable benefit/risk ratio applicable to medical treatment and is of a quantity that does not cause side effects. The level of the effective dose may be determined according to factors including the health condition of a patient, the type and severity of disease, drug activity, sensitivity to a drug, administration method, administration time, administration route and excretion rate, treatment duration, drugs combined or used simultaneously, and other factors well known in the medical field.

The composition may be administered by a method known in the art. The administration may be, for example, direct administration to a subject by any means or through a route such as intravenous, intramuscular, oral, transdermal, mucosal, intranasal, intratracheal or subcutaneous route. The administration may be systemic or local administration.

The pharmaceutical composition or cell therapeutic agent may be administered as an individual therapeutic agent or in combination with another therapeutic agent, may be administered sequentially or simultaneously with a conventional therapeutic agent, and may be administered in a single dose or multiple doses. **It** is important to administer an amount that can achieve the maximum effect with a minimal amount without side effects, considering all the above factors, and this may be readily determined by those of ordinary skill in the art.

A dosage of the pharmaceutical composition or cell therapeutic agent according to an embodiment may be in a range of 1.0 x 10³ to 1.0 x 10¹⁰ cells/kg (body weight) or subject, or 1.0 x 10⁷ to 1.0 x 10⁸ cells/kg (body weight) or subject, with respect to adherent cells. However, the dosage may be prescribed in various ways depending on factors such as formulation method, administration method, the age, body weight and gender of a patient, pathological condition, diet, administration time, administration route, excretion rate, and response sensitivity, and may be appropriately adjusted by those of ordinary skill in the art considering these factors. The number of administrations may be once or twice or more within the range of clinically acceptable side effects, and an administration site may be one or more sites. For animals other than humans, the same dosage per kg or per subject as for humans may be administered, or an amount calculated by converting the dosage according to, for example, the volume ratio (e.g., average value) of organs (such as the heart) between a target animal and a human may be administered. Examples of animals to be treated according to an embodiment may include humans and other target mammals, and specific examples thereof include humans, cats, dogs, monkeys, mice, rats, rabbits, sheep, cows, horses, pigs, and the like.

### Advantageous Effects of Invention

Cells according to an aspect and a composition including the same as an active ingredient have an increased IL-10 expression level without including a cryoprotectant, and thus can be effectively used for the prevention or treatment of an inflammatory disease or a renal disease.

### Brief Description of Drawings

FIG. 1 illustrates the degree of apoptosis in stem cells according to an embodiment (Fresh: negative control, cryoprotectant: positive control, Saline, Hartmann and Hartmann's Dex: experimental groups).
FIG. 2 illustrates the expression level of IL-10 in stem cells according to an embodiment (Viable Cells (Fresh): control, Apoptotic Cells (Hartmann's Dex): experimental group).
FIG. 3 illustrates *in vivo* changes in stem cells upon administration of stem cells according to an embodiment to mice (Viable Cells (Fresh): control, Apoptotic Cells (Hartmann's Dex): experimental group).
FIG. 4 illustrates changes of stem cells in internal organs upon administration of stem cells according to an embodiment to mice (Viable Cells (Fresh): control, Apoptotic Cells (Hartmann's Dex): experimental group).
FIG. 5 illustrates the recovery of mice upon administration of stem cells according to an embodiment to the mice (Model: negative control, Viable Cells (Fresh): positive control, Apoptotic Cells (Hartmann's Dex): experimental group).
FIG. 6 illustrates changes in renal morphology and changes in renal failure level upon administration of stem cells according to an embodiment to mice (Model: negative control, Viable Cells (Fresh): positive control, Apoptotic Cells (Hartmann's Dex): experimental group).

### Mode for the Invention

Hereinafter, preferred examples will be presented to aid in understanding of the present disclosure. However, these examples are provided only to facilitate the understanding of the present disclosure and are not intended to limit the scope of the present disclosure. Embodiments may allow for various modifications, and thus are not limited by the examples disclosed below and may be embodied in various forms.

### Example 1. Preparation of stem cells

Adult stem cells were prepared as follows. First, it was confirmed through an optical microscope that adult stem cells had proliferated to approximately 80 to 90 % of the bottom surface of a T75 flask, and then the culture medium was removed. 5 ml of Ca/Mg free DPBS was added to the T75 flask and then removed. 3 ml of Trypsin-EDTA (0.5%) was added and the T75 flask was shaken from side to side to spread the same evenly over the bottom surface. After incubating for 3 minutes in a 37 °C, 5 % CO₂ incubator, 10 ml of Ca/Mg free DPBS was added, the bottom surface was washed 3 to 5 times to collect the cells, which were then transferred to a 50 ml tube and centrifuged at 500 g for 5 minutes. After centrifugation, the cell pellet was confirmed, the supernatant was removed, and the cell pellet was released by tapping, and then suspended in Ca/Mg free DPBS. 10 µl of cell suspension was mixed with trypan blue and the total cell number was counted. After cell counting, the mixture was centrifuged at 500 g for 3 minutes, the cell pellet was confirmed, and the supernatant was removed.

Cryopreserved adult stem cells were prepared as follows. A cryoprotectant, saline, Hartmann's solution, and Hartmann's Dex solution (prepared so that 1.0 x 10⁶ cells were contained in 1 ml per vial) were each added to the cell pellet to suspend the cells. Then, 1 ml of the cell suspension was placed in each prepared 1.5 ml tube, the lid was closed, and the tubes were stored in an ultra-low temperature freezer at -80 °C or lower and cryopreserved for 1 month. The cryopreserved vials were thawed at 4 °C for 15 minutes and used in Experimental Examples 1, 2, and 3.

### Example 2. Induction of renal failure mouse model and administration of stem cells to mice

An animal model with induced renal failure was produced.

The renal failure mouse model was produced through induction using Cisplatin and lipopolysaccharide (LPS). Cisplatin (12 mg/kg) was administered intraperitoneally on Day 0 and 7, and LPS (5 mg/kg) was administered intraperitoneally on Day 1, 4, 8, and 11 to produce the model.

To confirm the therapeutic effect of the cells on renal failure, the cells were administered to the produced animal model.

Specifically, mice were placed and secured in a mouse restraint device, the tails of the mice were placed in warm water to dilate the blood vessels, and then the stem cells prepared according to Example 1 were administered intravenously to the tails of the mice by using an insulin syringe.

### Experimental Example 1. Confirmation of apoptosis of stem cells after cryopreservation using fluid

Apoptosis of the stem cells of Example 1 was examined. The degree of apoptosis was confirmed by analyzing Annexin V and propidium iodide (PI) values in non-cryopreserved adult stem cells (negative control), adult stem cells using a cryoprotectant (positive control), and adult stem cells without a cryoprotectant but using fluids (experimental groups).

The stem cells of Example 1 were prepared at 2 x 10⁵/100 µl and stained with Annexin V and PI at 4 °C for 15 minutes, followed by measurement using a flow cytometer.

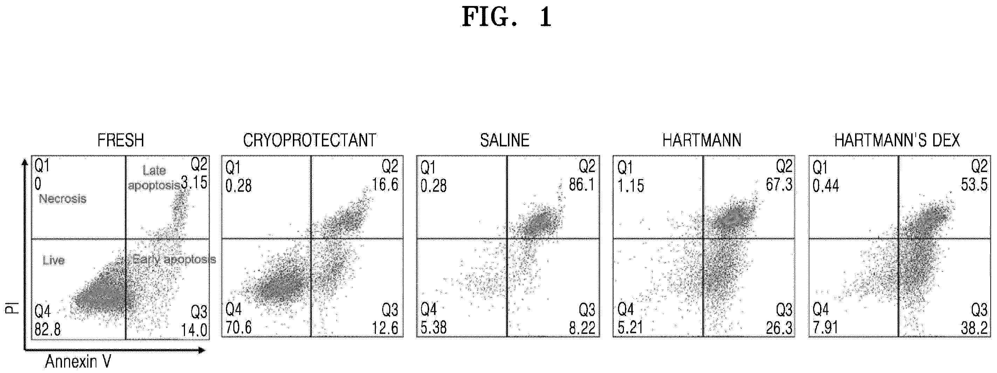

FIG. 1 illustrates the degree of apoptosis in stem cells according to an embodiment (Fresh: negative control, cryoprotectant: positive control, Saline, Hartmann and Hartmann's Dex: experimental groups).

As illustrated in FIG. 1, 80 % or greater of non-cryopreserved adult stem cells (negative control) survived, and 70 % or greater of cryopreserved adult stem cells (positive control) using a cryoprotectant survived after cryopreservation and thawing.

In contrast, in all of the experimental groups without a cryoprotectant and with fluids, according to an embodiment, 90 % or greater of cells were induced to undergo apoptosis. The highest late apoptosis was induced in saline, whereas the least late apoptosis and the highest early apoptosis were induced in Hartmann's Dex solution.

These results indicate that most of the stem cells without a cryoprotectant and with fluids, according to an embodiment, are induced to undergo apoptosis during cryopreservation, and among them, the case of using the Hartmann's Dex solution shows the greatest effect of inducing early apoptosis.

In the following examples and experimental examples, adult stem cells using Hartmann's Dex solution, which showed the greatest effect of inducing early apoptosis, were used as an experimental group.

### Experimental Example 2. Expression of IL-10 in fresh cells (viable cells) and Hartmann's Dex (apoptotic cells) during thawing after cryopreservation

The expression levels of IL-10 in the stem cells of Example 1 were examined. The expression levels of IL-10 in non-cryopreserved adult stem cells (control) and adult stem cells without a cryoprotectant and with Hartmann's Dex solution (experimental group) were analyzed.

One vial of each of the non-cryopreserved adult stem cells (control) and the adult stem cells using Hartmann's Dex solution (experimental group), prepared according to Example 1, was taken out and thawed at 4 °C, and the cells were separated using a centrifuge. The separated cells were lysed with a lysis buffer to prepare a protein mixture, and the protein concentration was measured. The same amount of protein mixture was subjected to sodium dodecyl sulfate (SDS) electrophoresis to separate proteins by size on a gel, and then transferred to a PVDF membrane. The membrane was allowed to react with antibodies against IL-10 and β-actin, each with a luminescent probe attached thereto, and bands were confirmed using a detection reagent. Subsequently, the IL-10 bands relative to the β-actin bands were quantified and the expression levels of IL-10 were compared.

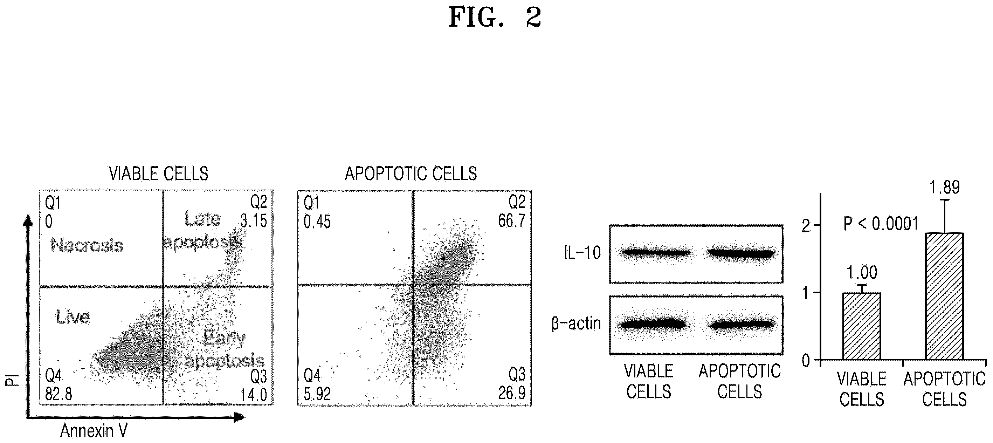

FIG. 2 illustrates the expression level of IL-10 in stem cells according to an embodiment (Viable Cells (Fresh): control, Apoptotic Cells (Hartmann's Dex): experimental group).

As illustrated in FIG. 2, the adult stem cells without a cryoprotectant and with Hartmann's Dex solution (experimental group) showed a statistically significant increase in IL-10 expression compared to the non-cryopreserved adult stem cells (control).

These results indicate that stem cells without a cryoprotectant and with fluid, according to an embodiment, show increased expression of IL-10 compared to non-cryopreserved stem cells.

This means that, compared to non-cryopreserved stem cells, stem cells without a cryoprotectant and using fluid, according to an embodiment, have a great effect of regulating the proliferation and differentiation of various immune cells such as T cells, B cells, NK cells, antigen-presenting cells, mast cells, and granulocytes, suppressing inflammatory responses, or modulating immune activation.

### Experimental Example 3. Confirmation of effect of apoptosis-induced stem cells on treatment of renal failure

### Experimental Example 3.1. Changes in distribution over time after in vivo transplantation

Changes in distribution over time after transplantation of the stem cells of Example 1 into mice were examined. For the non-cryopreserved adult stem cells (control) and the adult stem cells without a cryoprotectant and with Hartmann's Dex solution (experimental group), distribution of the stem cells in the bodies and internal organs of mice was examined for 24 hours after transplantation.

Specifically, the non-cryopreserved adult stem cells (control) and the adult stem cells without a cryoprotectant and with Hartmann's Dex solution (experimental group) were prepared using the method of Example 1.

The cells were stained using CellVue NIR815, which expresses GFP, and cell fluorescence was photographed using Pearl Trilogy equipment after transplantation of the cells. The cells were stained using NIR815 dye at a concentration of 2 x 10⁻⁶ M for 5 minutes before the Hartmann's solution of Example 1 was added, and then prepared for transplantation with Hartmann's solution.

The stem cells were transplanted using the method of Example 2. After transplantation, fluorescence in the bodies of mice and fluorescence in the internal organs of mice, such as the heart, lungs, liver, spleen, and kidneys, were measured using Pearl TRILOGY equipment.

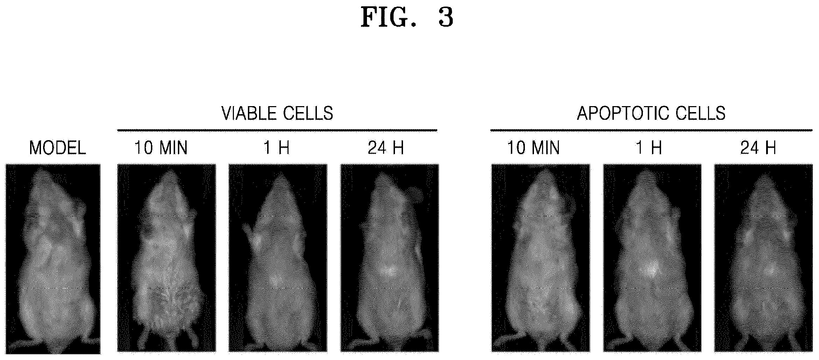

FIG. 3 illustrates *in vivo* changes in stem cells upon administration of stem cells according to an embodiment to mice (Viable Cells (Fresh): control, Apoptotic Cells (Hartmann's Dex): experimental group).

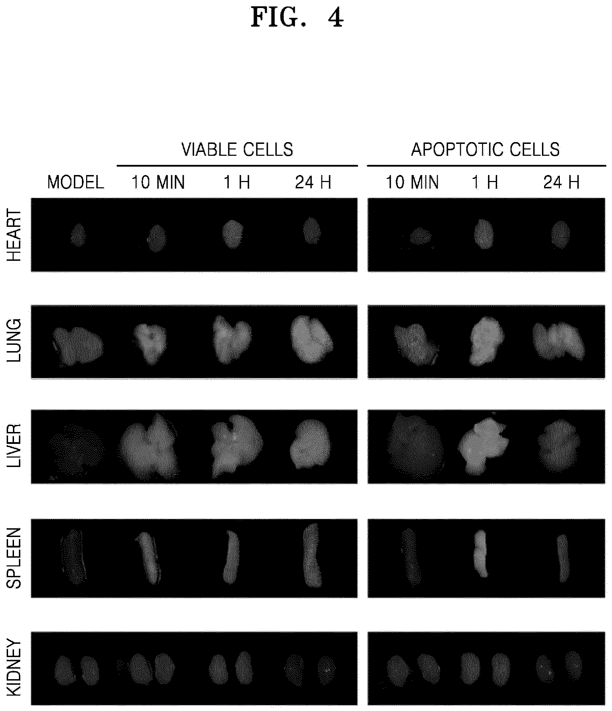

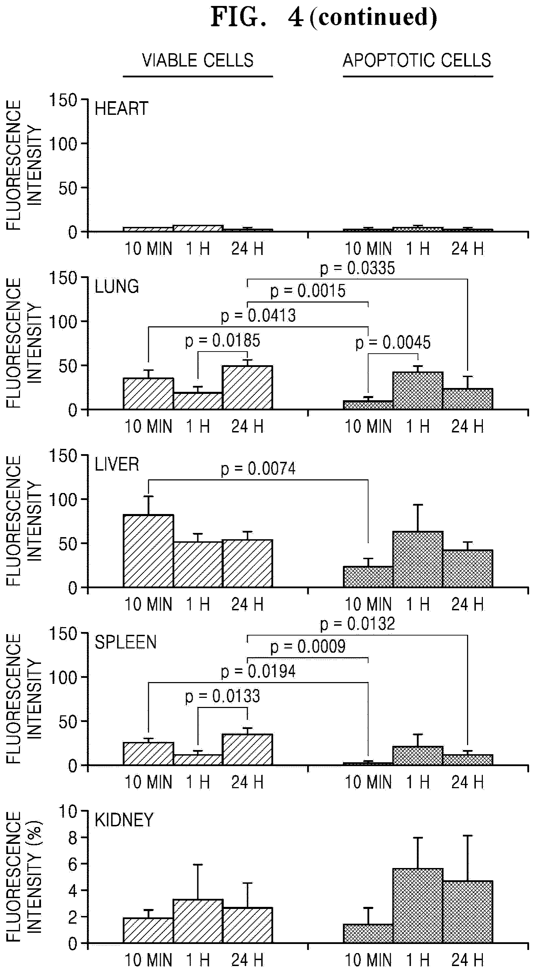

FIG. 4 illustrates changes of stem cells in internal organs upon administration of stem cells according to an embodiment to mice (Viable Cells (Fresh): control, Apoptotic Cells (Hartmann's Dex): experimental group).

As illustrated in FIG. 3, fluorescence expression in the bodies of mice was observed one hour after transplantation in both cases of the non-cryopreserved adult stem cells (control) and the adult stem cells without a cryoprotectant and with Hartmann's Dex solution (experimental group). In both groups, the fluorescence expression level was lower 24 hours after administration than that at 1 hour after administration. There was no significant difference in fluorescence expression level in the bodies of mice between the control and the experimental group.

As illustrated in FIG. 4, fluorescence in internal organs was most prominent in the liver for both the adult stem cells (control) and the adult stem cells without a cryoprotectant and with Hartmann's Dex solution (experimental group). In the kidneys, the fluorescence expression level was significantly higher in the experimental group than in the control. In the internal organs, there was no significant difference in fluorescence change pattern between the control and the experimental group.

These results indicate that the stem cells without a cryoprotectant and with fluid, according to an embodiment, show no difference in total amount change pattern and migration pattern of the stem cells after transplantation, compared to non-cryopreserved stem cells.

These results also indicate that the stem cells without a cryoprotectant and with fluid, according to an embodiment, have a greater therapeutic effect in the kidneys than non-cryopreserved stem cells.

This indicates that the stem cells without a cryoprotectant and with fluid, according to an embodiment, show no significant difference in cell migration, differentiation, cell death, treatment of inflammatory diseases, treatment of renal failure, or treatment of renal diseases, compared to non-cryopreserved stem cells.

### Experimental Example 3.2. Recovery of mice after transplantation into renal failure mouse model

The recovery of mice was examined after transplantation of the stem cells of Example 1 into the mice of Example 2.

Experiments for comparison in the body weight, renal weight, renal length, and BUN as an indicator of renal failure of mice were conducted on model mouse (negative control), the non-cryopreserved adult stem cells (positive control), and the adult stem cells without a cryoprotectant and with Hartmann's Dex solution (experimental group).

In addition, the kidneys of these three groups were analyzed histologically.

Specifically, the model mouse (negative control) was prepared using the method of Example 2, and the non-cryopreserved adult stem cells (positive control) and the adult stem cells without a cryoprotectant and with Hartmann's Dex solution (experimental group) were prepared using the method of Example 1.

The induction of the renal failure mouse model and administration of the stem cells to mice were performed using the method of Example 2.

The body weight, renal weight, renal length, and BUN of mice were measured using the following method. The body weight was measured using a scale, and mice were sacrificed using a CO₂ chamber. The abdomen of mice was opened with scissors, blood was collected from the femoral vein using a 1 cc syringe, and the kidneys were then extracted. The renal weight was measured using a microbalance, and the renal length was measured using a ruler.

The collected blood was centrifuged at 3000 rpm for 15 minutes, the plasma was transferred to a new tube, and BUN results were obtained using Accute equipment manufactured by Toshiba.

Histological analysis of the kidneys was performed using H&E and MT staining methods.

The kidneys were fixed for 3 days using 4 % paraformaldehyde, and then paraffin blocks were prepared. Subsequently, each paraffin block was cut to 4 µm and mounted on slides, and H&E staining was performed using hematoxylin and eosin dyes. Masson's Trichrome (MT) staining was performed by cutting the paraffin blocks to 4 µm, mounting on slides, and then staining with hematoxylin, Biebrich Scarlet-Acid fuchsin, and Aniline Blue dyes.

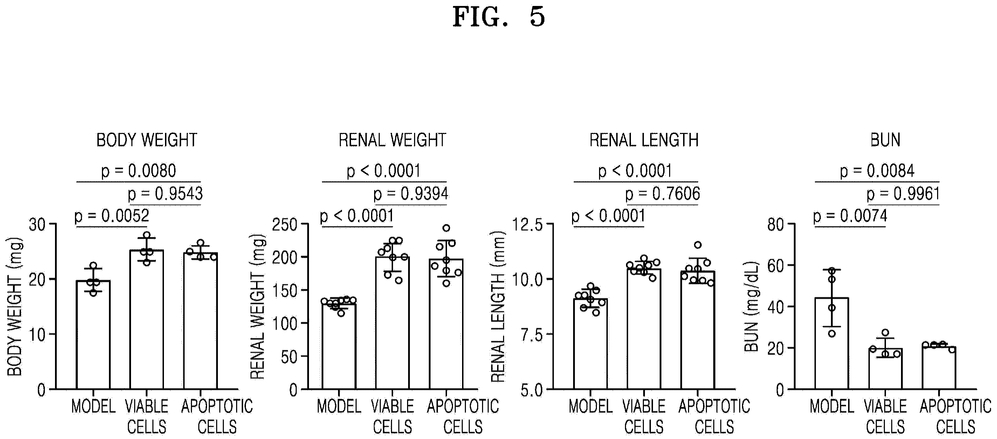

FIG. 5 illustrates the recovery of mice upon administration of stem cells according to an embodiment to the mice (Model: negative control, Viable Cells (Fresh): positive control, Apoptotic Cells (Hartmann's Dex): experimental group).

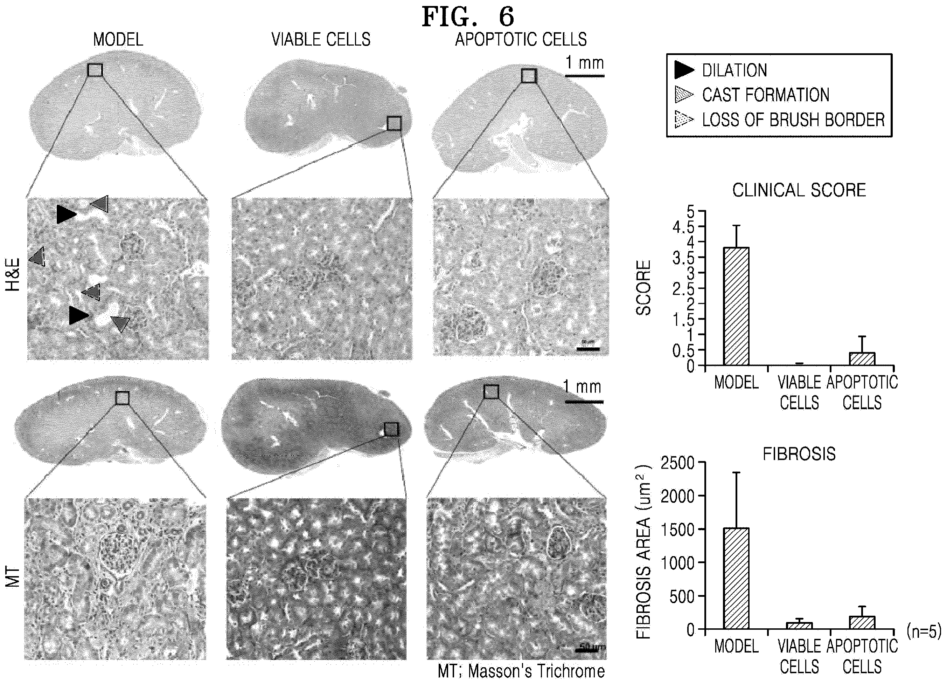

FIG. 6 illustrates changes in renal morphology and changes in renal failure level upon administration of stem cells according to an embodiment to mice (Model: negative control, Viable Cells (Fresh): positive control, Apoptotic Cells (Hartmann's Dex): experimental group).

As illustrated in FIG. 5, the body weight, renal weight, renal length and BUN of mice were recovered to normal levels after transplantation in both cases of the non-cryopreserved adult stem cells (positive control) and the adult stem cells without a cryoprotectant and with Hartmann's Dex solution (experimental group), compared to model mouse (negative control).

As illustrated in FIG. 6, kidney tissue and histological renal failure indices were restored to normal levels after transplantation in both the positive control and the experimental group, compared to the negative control.

In addition, there was no significant difference in recovery of these indices to normal levels between the positive control and the experimental group.

These results indicate that the stem cells without a cryoprotectant and with fluid, according to an embodiment, show no significant difference in the recovery of mice with renal failure after stem cell transplantation, compared to the non-cryopreserved stem cells.

This indicates that the stem cells without a cryoprotectant and with fluid, according to an embodiment, show no significant difference in the migration, differentiation and apoptosis of stem cells, treatment of inflammatory diseases, treatment of renal failure, or treatment of renal diseases., compared to the non-cryopreserved stem cells.

## Claims

1. An isolated apoptosis-induced cell that is induced to undergo apoptosis by cryopreservation and has a 1.5-fold or greater increase in interleukin-10 (IL-10) expression compared to the cell before cryopreservation.

2. The isolated apoptosis-induced cell of claim 1, wherein at least 75 % of an entire population is positive for Annexin V.

3. The cell of claim 1, wherein the cell is derived from any one selected from the group consisting of an adult stem cell, an induced pluripotent stem cell, and an embryonic stem cell.

4. The cell of claim 1, wherein the cell is derived from any one or more tissues selected from the group consisting of placenta, amniotic membrane, and umbilical cord.

5. The cell of claim 1, wherein a solution for the cryopreservation is a blood substitute.

6. The cell of claim 5, wherein the blood substitute comprises any one selected from the group consisting of: saline;
a solution comprising sodium chloride, potassium chloride, calcium chloride, and sodium lactate; and
a solution comprising sodium chloride, potassium chloride, calcium chloride, sodium lactate, and glucose.

7. The cell of claim 1, wherein the cell is induced to undergo apoptosis after thawing of the cryopreserved cell.

8. The cell of claim 1, wherein the cell is thawed at 0 to 5 °C for 10 minutes to 5 hours.

9. The isolated apoptosis-induced cell of claim 1, wherein the IL-10 expression is increased 2 to 2.5 times.

10. A pharmaceutical composition for the prevention or treatment of an inflammatory disease or a renal disease, comprising as an active ingredient: apoptosis-induced cells that are induced to undergo apoptosis by cryopreservation and have a 1.5-fold or greater increase in interleukin-10 (IL-10) expression compared to the cell before cryopreservation; or a culture solution of the cells.

11. The pharmaceutical composition of claim 10, wherein the inflammatory disease is selected from the group consisting of renal failure, nephritis, glomerulonephritis, gastritis, inflammatory bowel disease (IBD), hepatitis, inflammatory respiratory disease, gastric ulcer, irritable bowel syndrome, Behcet's disease, enteritis, Crohn's disease, asthma, ulcerative colitis, vasculitis, mucositis, stomatitis, peri-implantitis, periodontitis, pulpitis, gingivitis, pneumonia, dermatitis, atopic dermatitis, contact dermatitis, CREST syndrome, dermatitis herpetiformis, dermatomyositis, systemic scleroderma, erythema nodosum, Henoch-Schonlein purpura, Hidradenitis suppurativa, Lichen planus, Majeed syndrome, Schnitzler syndrome, psoriasis, eczema, acne, mouth ulcers, uveitis, pharyngitis, tonsillitis, otitis including otitis media, psoriatic arthritis, synovitis, meningitis, encephalitis, Bickerstaff's encephalitis, encephalomyelitis, spondylitis, osteomyelitis, Guillain-Barre syndrome, myelitis, neuromyelitis optica, cystitis, or acute inflammation at an infected site or wound site.

12. The pharmaceutical composition of claim 10, wherein the renal disease is any one or more selected from the group consisting of chronic renal failure, acute renal failure, glomerulonephritis, interstitial nephritis, diabetic nephropathy, glomerulosclerosis, renal fibrosis, Alport syndrome, IDDM nephritis, mesangial proliferative glomerulonephritis, proliferative glomerulonephritis, crescentic glomerulonephritis, renal interstitial fibrosis, focal segmental glomerulosclerosis, membranous nephropathy, minimal change disease, pauci-immune rapidly progressive glomerulonephritis, IgA nephropathy, polycystic kidney disease, Dent's disease, nephrocytinosis, Heyman nephritis, nephrotic syndrome, renal ischemia, podocytopathy or podocyte disease, proteinuria, preeclampsia, renal lesion, collagen vascular disease, benign orthostatic (postural) proteinuria, IgM nephropathy, aminoaciduria, Fanconi syndrome, hypertensive nephrosclerosis, hemoglobinuria, myoglobinuria, Wegener's granulomatosis, decreased glomerular filtration rate (GFR), renal arterial sclerosis, lupus nephritis, proximal tubular dysfunction, acute renal transplant rejection, chronic kidney transplant rejection, and non-IgA mesangial proliferative glomerulonephritis.

13. A method of preparing a composition containing apoptosis-induced cells, the method comprising:
preparing a cell-containing composition by immersing isolated cells in a solution containing a blood substitute;
cryopreserving the cell-containing composition to prepare the composition containing apoptosis-induced cells; and
thawing the composition containing apoptosis-induced cells.

14. The method of claim 13, wherein at least 75 % of an entire population of the cells in the preparation of the cell-containing composition is negative for Annexin V and PI, and at least 75 % of the entire population of the cells in the thawing is positive for Annexin V.

15. The method of claim 13, wherein the cells are derived from any one selected from the group consisting of adult stem cells, induced pluripotent stem cells, and embryonic stem cells.

16. The method of claim 13, wherein the adult stem cells are derived from any one or more tissues selected from the group consisting of placenta, amniotic membrane, and umbilical cord.

17. The method of claim 13, wherein the blood substitute comprises any one selected from the group consisting of:
saline;
a solution comprising sodium chloride, potassium chloride, calcium chloride, and sodium lactate; and
a solution comprising sodium chloride, potassium chloride, calcium chloride, sodium lactate, and glucose.

18. The method of claim 13, wherein the solution is a cryoprotectant-free solution.

19. The method of claim 13, wherein the cryopreservation is performed for 6 months or less.

20. The method of claim 13, wherein the thawing is performed at 0 to 5 °C for 10 minutes to 5 hours.

21. The method of claim 13, wherein the thawed cells have a 1.5- to 2.5-fold increase in IL-10 expression compared to the cell before cryopreservation.
